# EUROPÄISCHE PATENTANMELDUNG

(11) **EP 2 740 480 A1**
(43) Veröffentlichungstag der Anmeldung: **11.06.2014**
(21) Anmeldenummer: 12195980.3
(22) Anmeldetag: 06.12.2012
(51) Int. Cl.: A61K 33/00, A61P 3/00

(54) **Verfahren zur Verabreichung von hypoxischem und hyperoxischem Gas**

(71) Anmelder: Löffler, Bernd-Michael, 10623 Berlin (DE)
(72) Erfinder: Löffler, Bernd-Michael, Dr., 10623 Berlin (DE); Egorov, Egor, Dr., 10623 Berlin (DE); Maier, Sabine, 12103 Berlin (DE)
(74) Vertreter: Schubert, Klemens

(57) **Zusammenfassung**

Beschrieben wird ein Verfahren zur Verabreichung eines Gases an einen Menschen oder ein Säugetier, welches dadurch gekennzeichnet ist, dass in aufeinanderfolgenden Intervallen hypoxisches Gas alternierend mit hyperoxischem Gas verabreicht wird.

Beschrieben wir ferner die Verwendung des Verfahrens zur Leistungssteigerung, zur kosmetischen Gewichtsreduktion, zum Muskelaufbau, zur Verringerung oder Steigerung des Appetits, gegen Müdigkeit, zur Verlangsamung von Alterungsprozessen der Haut und/oder zur Verjüngung der Haut.

Das beschriebnene Verfahren wird als Intervall-Hypoxie-Hyperoxie-Therapie (IHHT) bezeichnet.

## Beschreibung

Die Erfindung betrfifft ein Verfahren zur Verabreichung eines Gases an einen Menschen oder ein Säugetier welches dadurch gekennzeichnet ist, dass in aufeinanderfolgenden Intervallen hypoxisches Gas alternierend mit hyperoxischem Gas verabreicht wird.

Es ist bekannt, dass das Inhalieren von sauerstoffarmer Luft zu einer vermehrten Bildung von Mitochondrien führt. Sportler nutzen diesen Effekt, indem sie bewusst in Höhen mit geringem Sauerstoffpartialdruck trainieren um ihre sportliche Leistungsfähigkeit zu verbesseren.

Bekannt ist auch die sogenannte hyperbare Sauerstofftherapie, bei der Luft mit einem hohen Anteil an Sauerrstoff verabreicht wird. In Dave K R et al.: "Hyperbaric oxygen therapy protects against mitochondrial dysfunction and delays onset of motor neuron disease in wobbler mice" wird beschrieben, dass die hyperbare Sauerstofftherapie mitochondriale Fehlfunktionen verbessert und einen positiven Einfluss auf den Energiestoffwechsel hat.

In WO 2011/051357 A1 wird ein Verfahren zur Behandlung mitochondrialer Dysfunktionen beschrieben, bei dem in mehreren aufeinanderfolgenden Intervallen abwechselnd sauerstoffarme und sauerstoffreiche Luft verabreicht wird. Gegenüber der reinen hyperbaren oder hypobaren Therapie hat die sog. Intervall-Hypoxie-Hyperoxie-Therapie (IHHT) den Vorteil, dass sie sehr viel effizienter ist als jede der beiden anderen Therapieformen für sich genommen.

Aufgabe der vorliegenden Erfindung ist es, die Intervall-Hypoxie-Hyperoxie-Therapie zu verbesseren und neue Anwendungsgebiete dafür zur Verfügung zu stellen.

Borreliose ist eine Bezeichnung für verschiedene Infektionskrankheiten, die durch Bakterien aus der Gruppe der Borrelien (Spirochäten) ausgelöst werden. Die Erkrankungen kommen beim Menschen und bei allen anderen Säugetieren vor und können durch den Befall aller Körpergewebe vielfältige klinische Symptome auslösen. Die Krankheit kann jedes Organ befallen, hauptsächlich sind jedoch das Nervensystem und die Gelenke betroffen. Die Übertragung der Erreger erfolgt hauptsächlich durch Zecken und Läuse, wodurch der Erreger in die Blutbahn gelangt und sich über die Blutgefäße im ganzen Körper ausbreitet. Nach der akuten Phase der Infektion, die sich durch grippeähnliche Symptome äußert, kommt es zu einem Befall der Organe, Gelenke, Muskeln oder des zentralen und peripheren Nervensystems. Die Folgen davon sind u.a. Gelenkentzündungen, neurologische Störungen , Herzprobleme, Gefäßentzündungen und Muskelschmerzen, die von Erschöpfungszuständen und chronischer Müdigkeit begleitet sein kann. Wird die Krankheit nicht rechtzeitig und ausreichend behandelt, so kann dies zur dauerhaften Schädigung der Organe führen.

Wegen der Möglichkeit des vielfachen Befalls verschiedener Organe ist die Behandlung der Borreliose im fortgeschritten Stadium sehr schwierig, da sich der Erreger nur kurze Zeit im Blut aufhält.

Eine Behandlung der Lyme Borreliose mit Antibiotika ist nur in der akuten Phase der Infektion mit einer klassischen Antibitotika Therapie erfolgreich. In der chronischen Phase der Borreliose, in der sich die Spirochäten aus dem Blut in die Zellen zurückgezogen haben, ist die Gabe von Antibiotika in den meisten Fällen wirkungslos. Das liegt daran, dass die meisten Antibiotika die Zellmembran nicht passieren können. Zwar gibt es auch zellmembran-gängige Antibiotika, diese haben jedoch den Nachteil, dass sie gleichzeitig auch die Mitochondrien der Zelle massiv schädigen.

Die Intervall-Hypoxie-Hyperoxie-Therapie ist eine Methode, bei der einer Person in mehreren aufeinanderfolgenden Intervallen sauerstoffreiche Luft (Hyperoxie) abwechselnd mit sauerstoffarmer (Hypoxie) Luft zum Atmen gegeben wird. Die IHHT ist ausführlich in der WO 2011/051357 A1 beschrieben. Der Sauerstoffgehalt in der Hypoxie-Phase beträgt gewöhnlich 9 bis 15 Vol-% und in der Hyperoxie-Phase gewöhnlich 30 bis 55 Vol-%.

IHHT ist ein Verfahren zur Verabreichung eines Gases an einen Menschen oder ein Säugetier, **dadurch gekennzeichnet, dass** in aufeinanderfolgenden Intervallen hypoxisches Gas alternierend mit hyperoxischem Gas verabreicht wird.

IHHT ist ferner **dadurch gekennzeichnet, dass** die Verabreichungsdauer des hypoxischen oder des hyperoxischen Gases 1 bis 20 Minuten, bevorzugt 2 bis 8 Minuten und besonders bevorzugt 3 bis 5 Minuten beträgt.

IHHT ist weiterhin **dadurch gekennzeichnet, dass** die Verabreichungsdauer von hypoxischem und hyperoxischem Gas unterschiedlich lang ist.

IHHT ist auch **dadurch gekennzeichnet, dass** die eine Sauerstoffkonzentration im hypoxischen Gas 9 bis 15 Vol-% und die Sauerstoffkonzentration im hyperoxischen Gas 30 bis 40 Vol-% beträgt.

IHHT ist ebenfalls **dadurch gekennzeichnet, dass** die Pause zwischen der Verabreichung des hypoxischen und des hyperoxischen Gases nicht mehr als 20 Minuten, bevorzugt weniger als 10 Minuten und besonders bevorzugt weniger als 5 Minuten beträgt.

IHHT ist auch **dadurch gekennzeichnet, dass** die Verabreichung in Einheiten erfolgt, die aus mehreren aufeinanderfolgenden Verabreichungsintervallen von hypoxischem/hyperoxischem Gas besteht.

IHHT ist weiterhin **dadurch gekennzeichnet, dass** 2 bis 10 Einheiten pro Woche verabreicht werden.

IHHT wirkt somit zur Leistungssteigerung, zur kosmetischen Gewichtsreduktion, zum Muskelaufbau, zur Verringerung oder Steigerung des Appetits, gegen Müdigkeit, zur Verlangsamung von Alterungsprozessen der Haut und/oder zur Verjüngung der Haut.

Die neuere Literatur zeigt, das Borrelien nur eine geringe Kapazität haben, sich vor reaktiven Sauerstoffverbindungen zu schützen [1-4]. Dies ist wahrscheinlich ein Grund, warum vorwiegend Körper-Kompartimente mit geringem Sauerstoff-Partialdruck befallen werden. Diese (z.B. Gelenke) sind als sogenannte bradytrophe Gewebe mit den klassischen Antibiotika am schlechtesten zu behandeln, weil sie nur wenige Kapillaren aufweisen und die Versorgung des Gewebes vor allem durch Diffusion der sie umgebenden Flüssigkeit erfolgt. Wegen dieses dominierenden "Diffusionsstoffwechsels" gelangen Antibiotika nur unzureichend an solche Gewebe.

Borrelien, insbesondere *Borrelia burgdorferi* sind empfindlich für oxidativen Stress [1-4]. Als oxidativer Stress wird eine Stoffwechsellage bezeichnet, bei der eine das physiologische Ausmaß überschreitende Menge hochreaktiver Sauerstoffverbindungen (ROS - reactive oxygen species) gebildet wird und/oder vorhanden ist. Diese reaktiven Sauerstoffverbindungen entstehen vor allem bei Stoffwechselvorgängen der mitochondrialen Elektronentransportkette und Cytochrom-P450-Oxidasen. Zu den ROS gehören hauptsächlich das Superoxid-Anionenradikal (O₂⁻), Wasserstoffperoxid (H₂O₂) und das Hydroxylradikal (OH).

Neben der Empfindlichkeit für oxidativen Stress weisen Borrelien einen Mangel an funktionellem Eisen auf [1] und sind deshalb empfindlich für die Fenton-Reaktion, worunter man die Reaktion von organischen Verbindungen mit Fe(II) und H₂O₂ zu Carbonyl- oder Hydroxylverbindungen versteht. Die IHHT stellt Hämoglobin sowohl gebunden als auch durch physikalische Lösung erhöhte Mengen Sauerstoff für die lokale Bildung von ROS zur Verfügung [5]. Dies führt einerseits zur Verbesserung der ROS-Stabilität der Körperzellen, und kann andererseits die Sauerstoff / ROS-Konzentration bradytropher Gewebe erhöhen. Dies ist eine mögliche Erklärung der reproduzierbar zu beobachtenden Verbesserung der Borreliose Symptomatik

Intervall-Hypoxie-Hyperoxie-Therapie verändert die intrazelluläre Sauerstoffkonzentration, wie auch die Sauerstoffkonzentration im Blutplasma in der hypoxischen Phase in Richtung einer Absenkung und in der hyperoxischen Phase in Richtung einer Anreicherung. Beide, Hypoxie und Hyperoxie, sind kausal für die Sauerstoff- und Stickstoff radikal (RNS) Produktion und beeinflussen die Replikation von *Borrelia Burgundii* [1,2]. Die IHHT stellt somit ein alternatives, nicht-invasives Verfahren zur Behandlung der reaktivierten chronischen Borreliose dar.

Eine abnormale dysregulierte NO Produktion ist die pathophysiologische Grundlage für eine sogenannte "stille" Entzündung ("Silent Infammation"). Darunter versteht man eine schleichenende Entzündung, die häufig zu chronischen Beschwerden führt. Die erhöhte NO-Produktion kann dann, bei Anwesenheit hoher Konzentrationen von Sauerstoffradikalen, zu einer hohen Peroxinitrit und damit Radikalstress Belastung werden. Die Höhe der dysregulierten NO Produktion lässt sich indirekt durch die Bestimmung von Citrullin im 1. Morgenurin messen. Eine erhöhte Citrullin Ausscheidung ist gleichzeitig beweisend für einen zellulären ATP Mangel.

Die Intervall-Hypoxie-Hyperoxie-Therapie reguliert auch den Stickstoff-Monoxid (NO) Stoffwechsel [8] und kann in Körperzellen zur Bildung von kurz- und langfristigen NO-Speichern s-Dinitrosothiol-Eisen-Komplexes (DNIC; Di-nitrosothiol-iron-complex) führen [9]. NO das in diese Speicherform überführt ist, steht nicht mehr für den Radikal-Angriff durch Sauerstoffradikale zur Verfügung. Dadurch kann die Bildung von reaktiven Stickstoffverbindungen gehemmt, und so die dadurch unterhaltene "stille" Entzündung reduziert werden. Die RNS Produktion kann über die Produktion von 3-nitrophenyl-Essigsäure (3-NPE) und die Entzündung durch die Bestimmung der Interleukine 6 und 8 bestimmt werden.

Mit IHHT kann der Zellstoffwechsel, wenn ausreichend funktionelles Eisen zur Verfügung steht, NO in kurz- und langfristige NO-Speicher überführen. Das so gespeicherte an DNIC's (Di-nitrosothiol-iron-complexes) gebundene NO steht nicht mehr für die Bildung von Peroxinitriten zur Verfügung. Dadurch kann mit einer IHHT der nitrosative Stress-Stoffwechsel unterbrochen und der intrazelluläre Inflammationsstoffwechsel normalisiert werden.

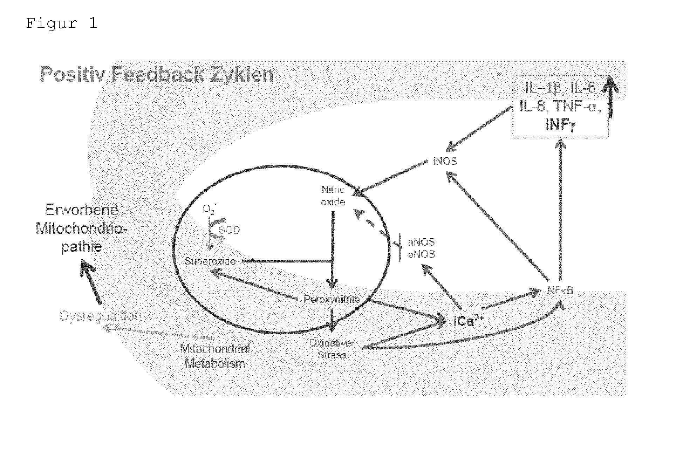

Oxidativer- und Nitrosativer Stress führen zu einer Erhöhung des intrazellulären Calciums (iCa²⁺), was den weiteren Anstieg von Entzündung vermittelnden Interleukinen zur Folge hat (Fig. 1). Dieser Interleukinanstieg führt zur erhöhten Expression der induzierbaren Stickstoffmonoxid Synthase (iNOS) und/oder der mitochondrialen Stickstoffmonoxid-Synthase (mtNOS). Dies wiederum hat eine weitere Erhöhung der intramitochondrialen NO Produktion zur Folge. Ein positiver Rückkopplungs-Zyklus ist entstanden. Positive Rückkopplungs-Zyklen sind im Gegensatz zu den negativ Rückkopplungs-Zyklen selbstverstärkend. Das überschüssig produzierte mitochondriale NO bindet unter den sauren mitochondrialen pH-Bedingungen irreversibel an Vitamin B12 an. NO-B12 und CN-B12 sind nicht mehr funktional und werden mit dem Urin ausgeschieden. Dies hat eine Verarmung der Mitochondrien an B12 zur Folge. Ohne B12 kann die Methyl-malonyl-CoA-Mutase keine ungradkettigen Fettsäuren und verzweigtkettige Aminosäuren mehr in den mitochondrialen Stoffwechsel einschleusen. Der mitochondriale Energie- und Aminosäurestoffwechsel wird schwer beeinträchtigt. Die im Stoffwechsel angestaute Methylmalonsäure wird mit dem Urin ausgeschieden. Schon relativ kleine Konzentrationen an Methylmalonsäure führen zu einer empfindlichen Störung des Zentralnervensystems.

Die mitochondrialen Stoffwechselstörungen, die durch ROS/RNS/NO-Stress erzeugt werden, können als erworbene mitochondriale Dysfunktion bezeichnet werden. Darunter ist eine durch ROS/RNS/NO Überproduktion entstandene Störung des mitochondrialen Stoffwechsels zu verstehen, der gleichzeitig mit einem Mangel bestimmter Vitamine (B12, B6, B3, B1, K2), einer Reduktion von NAD+/NADP+ und zellulären/mitochondrialen Mineralstoffdefiziten/-ungleichgewichten (K, Ca, Mg, Zn, Fe, Se) einhergeht. Die Leitsymptome einer erworbenen mitochondrialen Dysfunktion sind: Kurze Essintervalle, postprandiale Müdigkeit, Alkoholintoleranz, Durchschlafstörungen(Schwitzen, Herzjagen, Apnoe, Angstträume, Nykturie, Bruxismus), unerholsamer Schlaf trotz ausreichender Schlaflänge, morgendlich lange Anlaufzeit, Gelenksteife, Lendenwirbelsäulen(LWS)-Schmerzen, morgendliche Inappetenz, "Fress"-Attacken auf Süßes und Kohlenhydrate, die sich zum Nachmittag hin verstärken.

Laborparametrisch findet sich immer eine gestörte Ca²⁺ Homöostase (Calcitriol [pg/ml]/Calcidiol [ng/ml] Ratio > 2), Methylmalonsäure Ausscheidung im 1. Morgenurin > 0,4 mg/g Kreatinin, Homocystein > 7 mol/1. Ein Training mit IHHT erhöht, ohne Ausgleich defizienter Vitalstoffe (z.B. Vitamin D, Calcium, Magnesiun, Vitamin B12, Folsäure, Vitamin B1, B2, Vitamin C) die intrazelluäare Fähigkeit zur B12 Synthese.

Mit Hilfe der IHHT kann der mitochondriale Stoffwechsel wesentlich erhöht und der bestehende nitrosative Stress, d.h. die geschilderte positive Rückkopplung unterbrochen werden.

Die Intervall-Hypoxie-Hyperoxie-Therapie kann ferner über die Regulierung des hypoxic-inducible-factor-1-a (HIF-1a) die Expression der Enzyme der Glykolyse, welche den Abbau von Kohlenhydraten zu Pyruvat unter Energiegewinnung beschreibt, beeinflussen. Durch die IHHT kann somit direkt in den Stoffwechsel eingegriffen werden. Beispielsweise kann die IHHT durch eine Erhöhung des Stoffwechsels unterstützend bei der Gewichtsreduktion wirken oder zur Erhöhung der sportlichen Leistung dienen.

Mit der Intervall-Hypoxie-Hyperoxie-Therapie kann auch die Regulation des Hypoxic-Inducible-Factor-1-α den glykolytischen Stoffwechsel sowie die Insulin-Resistenz über die Regulation der Glukose-Transporter 1 und 3 beeinflusst werden [6, 7].

Zusammenfassend lässt sich feststellen, dass mit der IHT eine Vielzahl von medizinischen und nicht-medizinischen Anwendungsmöglichkeiten denkbar sind. Die IHHT kann beispielsweise angewandt werden bei:
- Hypertonie, insbesondere introgene Hypertonie
- Übergewicht
- Hypercholesterinämie
- Hypertriglyceridämie
- Dyslipädiemie
- COPD und andere obstruktive Lungenerkrankungen. Verbesserung der Vitalkapazität, usw.
- Chronische "silent" Inflammation (NO und RNS getriebene chronische Entzündung)
- Typ-2-Diabetes, Insulinresistenz
- Senkung des HbA1c
- Senkung des "Süßhungers"
- Zuckerstoffwechselstörungen
- Behandlung der Lactacidose
- Umprogrammierung des Citrat-Cyclus (Auch Krebs- oder Tricarbonsäure Cyclus) mit spezifischer Senkung von Citrat und cis-Aconitsäure und Anhebung von a-Ketoglutarsäure
- Therapie des Pyruvat-Stoffwechsels: Verbesserung der Ökonomie, geringere Pyruvat Ausscheidung
- Für anabole Stoffwechsel Umstellung: Muskelaufbau (Leistungssteigerung, Gewichtsnormalisierung)
- Für Lipolyse, Einleitung und Unterstützung der Lipolyse (Gewichtsnormalisierung)
- Verbesserung der Herz-Raten-Variabilität: RMSSD, E/I Quotient, E-I Differenz
- Aktivierung des parasymphatischen Systems
- Verbesserung des mitochondrialen Membran Potentials (Parameter einer mitochondrialen Regeneration
- Mitochondriale Regeneration
- Verbesserung der mitochondrialen ATP Synthese
- Senkung der metabolischen Acidose
- Verbesserung / Entlastung des Leber Stoffwechsels
- Verlangsamung des Alterungsprozesses der Haut/Verjüngung der Haut
- Müdigkeit
- Verringerung, bzw. Steigerung des Appetits
- Verbesserung des allgemeinen Wohlbefindens

Es hat sich gezeigt, dass durch die gezielte Veränderung einzelner Parameter bei der IHHT-Behandlung der Stoffwechsel gezielt beeinflusst werden kann, wodurch unterschiedliche Beschwerden gezielt behandelt werden können.

Zu den Parametern, die variiert werden können, gehören beispielsweise der Sauerstoffgehalt der hypoxischen, bzw. hyperoxischen Phase, die Dauer der Verabreichung des Gases, die Anzahl der Zyklen, in denen hypoxisches / hyperoxisches Gas nacheinander verabreicht werden sowie die Frequenz der Behandlungseinheiten.

Typischerweise beträgt die Sauerstoffkonzentration im hypoxischen Gas 9 bis 15 Vol-% und die Sauerstoffkonzentration im hyperoxischen Gas 30 bis 55 Vol-% beträgt.

Die Verabreichungsdauer kann 1 bis 20 Minuten betragen. Bevorzugt sind jedoch kürzere Verabreichungseinheiten zwischen 2 und 8 Minuten oder 3 bis 5 Minuten. Die Verabreichungsdauer der hypoxischen Phase muss nicht gleich sein mit der der hyperoxischen Phase. Je nachdem, welcher Effekt erzielt werden soll, kann die hyoxische Phase kürzer oder länger sein als die hyperoxische Phase. Durch die unterschiedliche Dauer können verschiedene Stoffwechselvorgänge gezielt angeregt oder verlangsamt werden.

Hypoxisches und hyperoxisches Gas werden hintereinander verabreicht, wobei bevorzugt ist, dass die Verabreichung unmittelbar hintereinander erfolgt, so dass keine längeren Pausen entstehen. Der Zeitraum zwischen der Verabreichung von hypoxischem und hyperoxischem Gas sollte möglichst kurz sein und nicht mehr als 20 Minuten betragen. Bevorzugt sind Pausen von weniger als 10 Minuten.

Ein IHHT-Behandlungszyklus besteht aus einer Verabreichungseinheit eines hypoxischen und einer Verabreichungseinheit eines hyperoxischen Gases. Eine Behandlung kann aus einem oder mehreren Behandlungszyklen bestehen. Gewöhnlich werden, je nach Anwendung, 2 bis 10 Behandlungszyklen pro Behandlung durchgeführt, wobei die Behandlungszyklen bevorzugt unmittelbar hintereinander erfolgen.

Eine Therapie kann aus mehreren einzelnen Behandlungen bestehen, die ein oder mehrmals in der Woche stattfinden können. Je nach Anwendung kann die Gesamt-Terapiedauer variieren und kann zwischen 1 Tag und mehren Monaten liegen. Bei längeren Therapien kann während der Therapiedauer die Behandlungsparameter verändert und den erzielten Fortschritten angepasst werden.

Die Erfindung wird anhand der folgenden Ausführungsbeispiele näher erläutert.

### 1. Verwendung der IHHT-Therapie zur Behandlung von Borreliose.

Zwanzig Patienten mit einer reaktivierten chronischen Borreliose wurden mit verschiedenen Protokollen einer Hypoxia-Hyperoxia-Interval-Therapie behandelt. Dabei wurde in der Hypoxie-Phase sauerstoffreduzierte Atemluft mit 9-15 Vol-% O₂ und in der Hyperoxie-Phase sauerstoffangereicherte Luft mit 30-40 Vol-% Sauerstoff geatmet. Verschiedene Zeit-Intervalle wurden exploratorisch untersucht. Die besten Ergebnisse wurden mit einem Protokoll von 1 Minute Hypoxie gefolgt von 9 Minuten Hyperoxie erreicht. Die Patienten wurden für jeweils 60 Minuten (6 Behandlungszyklen) pro Anwendung therapiert. Die Behandlungsintervalle lagen zwischen täglich einer IHHT Einheit und 2 IHHT Einheiten pro Woche. Die Therapiedauer ersteckte sich von 10 bis 20 Therapie-Einheiten, je nach Schweregrad der Symptomatik. Vor und nach der Therapie wurde ein Borrelien IgG/IgM ELISA-Screen, Borrelien recom Bead-Test (Blot), ein LTT-Borrelien (Lymphozyten Transformations-Test) durchgeführt.

Während der ersten 1-5 Behandlungseinheiten verschwanden bei allen Patienten die typischen Beschwerdesymptome (Nerven-, Muskel- und Gelenkschmerzen, Schlafstörungen, Abgeschlagenheit, usw.).

Im Fall einer zum Zeitpunkt der Therapie fünfzigjährigen Frau bestand eine chronische Borreliose (ED Februar 2001), Antibiose 2001, 2003 und 2009. Seit 2008 litt die Frau unter starken, wandernden Muskel- und Gelenkschmerzen, die Arme konnten nicht mehr gehoben werden, die Frau hatte starke allgemeine Leistungseinschränkung und litt unter chronischer Müdigkeit und Schlafstörungen. Seit Frühjahr 2009 war An- und Ausziehen der Kleidung nur noch mit Hilfe möglich. Die LTT von 04/2009 und 03/2010 (vor Beginn der IHHT Therapie) sind identisch und zeigen eine reaktive Lyme Borreliose. Von 04/2010 bis 06/2010 wurde die IHHT (3 Behandlungseinheiten pro Woche a 60 Minuten, insgesamt 20 Einheiten) durchgeführt. Die Symptomatik reduzierte sich ab der 3. Behandlung bis zum völligen Verschwinden. Das Kontroll-LTT von 09/2010 war negativ. Bis zum Dezember 2010 bestand ein komplett Symptom freies Intervall.

### 2. Einfluss der IHHT auf den Bodymass-Index.

Eine 21-jährige Patientin mit schwerer Erschöpfungssymptomatik, die in der Anamnese eine Gewichtszunahme von 12 kg innerhalb von 12 Monaten angab, ohne dass sich ihr Ernährungsverhalten deutlich geändert hatte, wurde über 6 Monate mit IHHT (2x wöchentlich, morgens nüchtern, jeweils 45 Minuten) behandelt. Bei der Patientin wurde vor der Behandlung eine Insulinresistenz festgestellt. Der ATP (Adenosintriphosphat)-Wert betrug 0,44 µM. Mit der Patientin wurde weder eine Diät, noch Lebensstilverändernde Maßnahmen vereinbart.

Drei Monate vor und sechs Monate nach Beginn der Therapie wurden bei der Patientin die folgenden Laborparameter bestimmt: Basis Labor (Kleines Blutbild) Calcidiol, Lactat/Pyruvat, ATP, Insulin, 3-Nitrophenyl-Essigsäure, Citrullin, Methyl-Malonsäure. Zusätzlich wurde die Körper-Zusammensetzung mit einer Body-Impedanz Messung (BIA) zu Beginn sowie fünfmal im Verlauf und einmal in der Nachbeobachtung gemessen. Ferner wurde die Herzraten-Variabilität (HRV) vor und nach der IHHT bestimmt. Aus dem im nüchternen Zustand bestimmten Insulin- und Blutzuckerwert wurde der HOMA-Index (Homöostasis-Model-Assessment) berechnet.

Die Patientin wurde 2 x pro Woche morgens nüchtern mit einem IHHT Protokoll von 5 Minuten Hypoxie mit einem Hypoxic-Training-Index von 87,7 (?) gefolgt von 3 Minuten Hyperoxie im Biofeedback Modus (BFM), insgesamt 29 mal über einen Zeitraum von 5 Monaten behandelt. Dabei wurde die Intensität von ursprünglich 88% SpO2 BFM auf 81% BFM gesteigert. Die bei der Labor Untersuchung festgestellten Vitalstoffmängel wurden parallel zur Therapie ausgeglichen.

### Ergebnis:

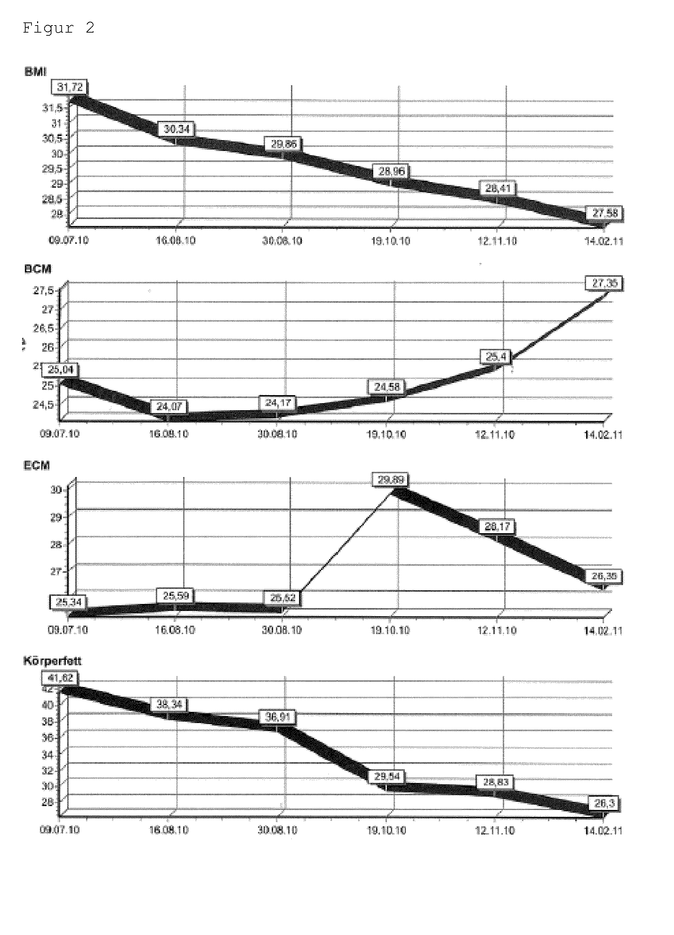

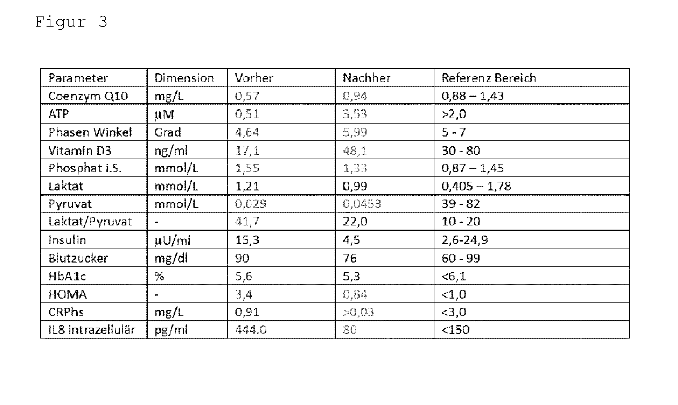

Nach Beendigung der Therapie wurde der Bodymass-Index (BMI) um 4,14 kg/m2 reduziert. Die Körperzellmasse (BCM) stieg Brutto um 2,58 kg. Körperfett sank um 15,32 kg. Das Extrazellulärvolumen (ECM) stieg um 1,01 kg. Unter Berücksichtigung, dass 1 kg Körperfett einer Zellmasse von 150 g entspricht, erhöhte sich das BCM um 4,83 kg. In der fast dreimonatigen Nachbeobachtungsphase blieb das Gewicht stabil. Körperfett stieg leicht um 0,3 kg, während das ECM um 0,77 kg fast auf das Ausgangsvolumen fiel (Fig. 2). Das HRV wurde über die Behandlung deutlich verbessert, insbesondere die E-I Differenz, mittlere Herzfrequenz, RMSSD, und Pulswellen Latenzzeit ändern sich durch die IHHT signifikant im Sinne einer parasympathischen Aktivierung (Fig. 3).

Die vor Behandlungsbeginn bestehende Insulinresistenz, konnte korrigiert werden (Fig. 3), der permanente Süßhunger verschwand während der Behandlung.

Über einen Zeitraum von 6 Monaten konnte das Gewicht und die Leistungsfähigkeit der Probandin signifikant verbessert werden. Eine anfänglich bestehende Insulinresistenz wurde ebenso normalisiert wie die Symphatico-Parasymphaticotone Balance. In der ca. dreimonatigen Nachbeobachtungsphase blieben das Gewicht und die sonstigen Vitalleistungen stabil.

Die Patientin ist ein typisches Beispiel für die massiven Vitalstoffdefizite, die zu schweren Stoffwechsel-und Leistungsstörungen führen können. Die IHHT stellt eine sehr effiziente Methode zur Korrektur dieser Stoffwechsel-Dysfunktion über die gezielte Ansteuerung von HIF-1a und Nrf-2 dar.

### 3. Einfluss der IHHT auf die mitochondriale Stoffwechselaktivität.

Eine Gruppe von 19 Männern und Frauen, mittleres Alter 51,0 (Standardabweichung 10,3 Jahre) durchlief innerhalb von 3 Wochen 10 IHHT Einheiten von 35 Minuten Dauer (5 Min. Hypoxie, Baseline PcO₂ (Sauerstoffpartialdruck) 80%; 2 Min Hyperoxie 38% 02). Die Teilnehmer wurden nach der Eingangsuntersuchung in einer Doppelblindstudie in 2 Gruppen aufgeteilt: 10 Verum, 9 Kontrollen. In der Kontrollgruppe wurde normale Raumluft geatmet.

Zu Beginn und am Ende der Studie wurden folgende Messwerte erhoben: Großes Labor (Großes Blutbild), Plasma Q10, Nitrophenylessigsäure, Methylmalonsäure, Citrullin, mitochondriale Aktivität, forcierte 1-Minuten Herzratenvariabilität (HRV), Body-Impendanz Messung.

Die Datenauswertung erfolgte mittels paired /unpaired T-Test. In der Kontrollgruppe gab es keine signifikanten Veränderungen. In der Verum-Gruppe stieg nach 10 Behandlungen der Hb (g/1) 14,3+1,3 (Mittelwert + Standardabweichung) vs. 14,8+1,3 p=0.01; sanken TgL mg/dl 151,3+76,8 vs 124,5+54,7 p=0,07; Bauchumfang (cm) 95,5+17,7 vs. 93,3+17,3 p=0.009; Bodymassindex (kg/m2) 26,6+5,1 vs 26,3+4,8 p=0.07.

Die mitochondrialen Stoffwechselparameter Coenzym Q10 (mg/l) 1,0+0,3 vs. 1,4+0,3 p<0,0001 und mitochondriale Aktivität (%) 84,7+6,6 vs. 94,6+4,3 p=0,004 stiegen signifikant. Die HRV Parameter: E-I Differenz (1/min) 14,4+8,1 vs. 18,1+7,2 p=0,02; E/I Quotient 1,2+0,1 vs. 1,4+0,1 p<0,001 und RMSSD (ms) 59,3+38,7 vs. 94,1+25.9 p=0.02 verbesserten sich signifikant. In der gesamten Gruppe der Probanden zeigte sich eine signifikante inverse Korrelation zwischen Q10 und Methylmalonsäureausscheidung im 1. Morgenurin, als Maß für einen funktionellen mitochondrialen B12 Mangel: MMS= -1,18Q10 + 1,73 r2=0,304. IHHT ist ein effektives und sicheres Verfahren, um die mitochondriale Stoffwechselaktivität schon nach kurzer Anwendung signifikant zu steigern. Daraus kann gefolgert werden, dass körpereigenes Q10 ohne Substitution gesteigert werden kann. Q10 ist von zentraler Bedeutung für den mitochondrialen Energie und Oxidationsstoffwechsel, wie auch für die Krebsprävention.

### 4. Einfluss der IHHT auf den mitochondrialen Stoffwechsel und die Herzraten-Variabilität.

24 Männer und Frauen wurden für eine IHHT-Studie laborparametrisch untersucht. Die Probanden wurden in zwei Gruppen, eine Behandlungsgruppe und eine Placebogruppe unterteilt.

Gemessen wurde das große Standard Labor (Blutbild, Blutzucker, Lipidstatus, mitochondriale Aktivität, Coenzym Q10, Nitrophenyl Essigsäure (Nitrosativer Stress), Citrullin (mitochondrialer Arginistoffwechsel), Methylmalonsäure (mitochondrialer B12 Status)) sowie Körpermasse, Größe und Gewicht der Probanden. Ferner wurde eine Body-Impendanz-Analyse durchgeführt und die forcierte 1-Minuten Herzratenvariabilität gemessen.

Die Behandlungsgruppe wurde in 3 Wochen 10x mit jeweils einem 35 minütigen IHHT Training (5 Minuten Hypoxie gefolgt von 2 Minuten Hyperoxie, 5 Trainingszyklen) im BioFeedback Modus an einem One-Plus Hypoxicator (arterielle Ziel-Sauerstoffsättigung 80% SpO2) behandelt. Die Placebogruppe erhielt ebenfalls ein Training an einem One-Plus Hypoxicator, allerdings atmeten diese Probanden die ganze Zeit über Raumluft. Die Trainingsabstände zwischen den einzelnen Traingseinheiten variierten zwischen 1 bis 4 Tagen, im Mittel 2 Tage. 1 Woche nach Abschluß des Trainings wurden alle Labor- und Vitalwerte ein zweites Mal bestimmt. Die Probanden wußten nicht, ob sie in der Verum- oder der Placebo-Gruppe eingeteilt waren.

### Ergebnisse:

Von den 24 Probanden beendeten 10 die Studie in der Verum-Gruppe und 8 Teilnehmer in der Placebo-Gruppe. Veränderungen der Messparameter wurden in den Gruppen mittels two-tailed-paired T-Test, zwischen den Gruppen mittels two-tailed-unpaired T-Test ermittelt.

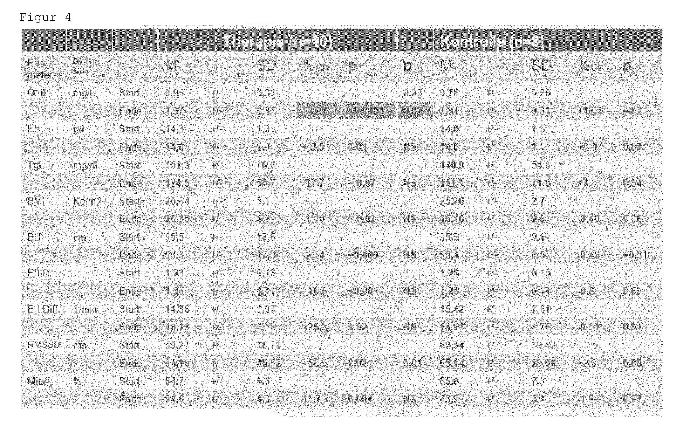

In der Behandlungsgruppe berichteten alle Teilnehmer über eine über die Trainingszeit deutlich verbesserte allgemeine Leistungsfähigkeit, besseren Schlaf und weniger "Zucker-Hunger". Bei den Probanden war ein unterschiedlich ausgeprägtes mitochondriales B12-Defizit mit dementsprechend erhöhter Ausscheidung von Methylmalonsäure(MMS) und reduziertem Q10 Serum Spiegel zu beobachten.
Es zeigte sich des Weiteren ein unterschiedlich stark ausgeprägter nitrosativer Stress, quantifiziert mit der Nitrophenylessigsäure (NPE)-Ausscheidung im 1. Morgenurin. Zwischen NPE und MMS Ausscheidung zeigte sich eine signifikante Korrelation, ebenso zeigte sich eine inverse Korrelation zwischen MMS und den Serum Q10 Spiegeln. In der Herzratenvariabilität (HRV) zeigte sich eine signifikante Aktivierung des Parasymphatischen Nervensystems mit signifikant verbesserter RMSSD und E-I Differenz. Das IHHT Training konnte den Q10 Serum Spiegel signifikant verbessern (Fig. 4). Parallel dazu verbesserten sich entscheidende Vital-Parameter (Fig. 4).

Die durchgeführte Studie zeigt, dass mit schon 10 IHHT Trainingseinheiten, selbst ohne Substitution defizitärer Vitalstoffe, die mitochondriale Energiegewinnung wesentlich verbessert und der bestehende nitrosative Stress reduziert, d.h. der NO-ONOO positiv-Feedback-Zyklus unterbrochen werden kann.

### 5. Einfluss der IHHT bei der Behandlung von Multipler Sklerose.

Ein Patient mit schubförmiger MS wurde über 6 Monate mit einer oralen antiinflammatorischen orthomolekularen Substitutionstherapie behandelt. Unter dieser Therapie konnte die chronische Inflammation nicht verbessert werden. Im Gegenteil stieg sogar die Menge an zirkulierenden proinflammatorischen Zytokinen. Eine IHHT Behandlung mit 20 IHHT Einheiten mit eskalierendem hypoxischem Reiz (5 bis 8 Minuten Hypoxie, Biofeedback 88% -80% SpO₂, 150 - 250 HTI Einheiten (HTI = Hypoxic-Training-Index) wurde über 10 Wochen durchgeführt (2 Trainingseinheiten pro Woche). Damit konnte die Produktion von zirkulierenden proinflammatorischen Zytokinen signifikant gesenkt werden. Der 3-NPE-Spiegel wurde auf 0 reduziert und die Interleukin 6- und 8-Produktion normalisiert. Eine Nachmessung nach 6 Monaten ergab ein wesentlich normalisiertes Interleukin-Profil.

### 6. Einsatz der IHHT bei der Behandlung von Mundboden Carzinom.

Eine Patientin mit inoperablem Mundboden-Carcinom zeigte auch nach einer 3x wöchentlich durchgeführten antioxidativen Infusionstherapie mit Hochdosis Vitamin C Infusionen (75g Vitamin C pro Infusion) einen massiven nitrosativen Stress mit einem massiv dysregulierten NO/Arginin-Stoffwechsel.

Eine zusätzlich zur Infusionstherapie durchgeführte IHHT Behandlung wie in Beispiel 5 mit eskalierendem hypoxischen Reiz konnte den nitrosativen und NO/Arginin-Stoffwechsel, wie auch den mitochondrialen B12 Stoffwechsel komplett normalisieren.

### 7. Reprogrammierung des motochondrial-Stoffwechsels und motochondriale Regeneration mit IHHT.

Eine fünzigjährige Probandin ohne Beschwerden, Alter 50 Jahre, BMI 21 kg/m2 wurde in 4 Wochen (3 Trainingseinheiten pro Woche) mit IHHT Einheiten von 45 Minuten Dauer: 5 Minuten Hypoxie 3 Minuten Hyperoxie, Biofeedback 80% SpO2 ohne Zugabe von Vitalstoffen trainiert. Labor- und Vital-Parameter wurden vor und nach dem Training bestimmt.

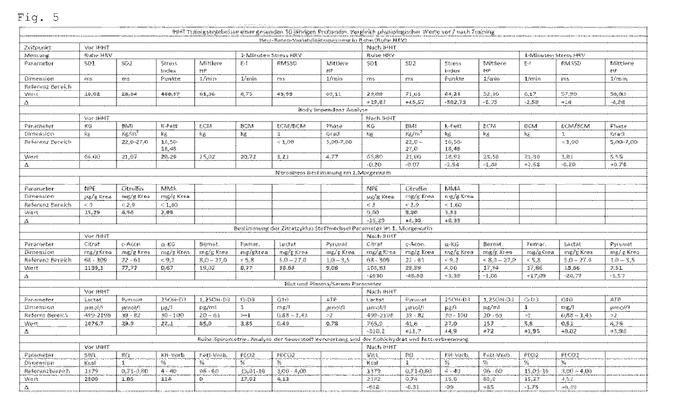

Laktat/Pyruvat und Adenosin-Triphospat (ATP) im Blut (nüchtern), die Metaboliten des Citrat-Cyclus in 1. Morgenurin, die Körperzusammensetzung (Körperfett, Körperzellmasse, Körperwasser), der Energiehaushalt und die Energiequelle (Kohlenhydrate oder Fett) mit einer Ruhe-Respirometrie wurde vor und nach Behandlung der IHHT Training Einheiten gemessen. Die Ergebnisse sind in Fig. 5 zusammengefasst.

### Ergebnis:

Bei unveränderten BMI von 21 kg/m2 baute die Probandin 1,4 kg Fett ab und 2,4 kg Zellmasse auf, das Körperwasser sank um 1 kg. Die ATP Produktion stieg von 0,78 auf 4,76 M. Lactat fiel von 1,08 auf 0,77 mol/l, Pyruvat stieg von 30 auf 42 mol/l. Citrat im 1. Morgenurin fiel von 1139 auf 109 mg/g Kreatinin. Der Gesamtenergieumsatz in Ruhe fiel von 2800 auf 2182 kcal. Die Kohlehydratverbrennung fiel von 100 auf 15%. Konsekutiv stieg die Fettverbrennung von 0 auf 85%. Aus den Körperzusammensetzungs- und Stoffwechseldaten der Probandin läßt sich auf eine deutliche Aktivierung des mitochondrialen Energiestoffwechsels schließen, der über einen Behandlungszeitraum von 6 Wochen zu einer positiven Veränderung der Körperzusammensetzung geführt hat.

Der mitochondriale Citrat-Cyclus und der EnergieStoffwechsel konnte durch das Training normalisiert werde. Der zu Beginn des Trainings bestehende massive nitrosative Stress wurde auf 0 reduziert. Weil keine B12- und Vitamin D-Substitution erfolgte, verschlechterten sich die entsprechenden Stoffwechsel-Parameter.

Der zu Beginn der Behandlung bestehende rein glycolytische Energiestoffwechsel konnte in eine dominierende Fettverbrennung gedreht werden. Gleichzeitig wurde der anabole Aminosäure-Stoffwechsel normalisiert. Dadurch konnte die Probandin in 4 Wochen 2,4 kg Muskelmasse auf-und 1,4 kg Fett abbauen. Als "Nebeneffekt" wurde das Hautbild der Probandin durch eine parallele Behandlung mit der ageLine One® Cosmeceutical Serie signifikant verjüngt.

### < Literaturverzeichnis >

1) Boylan Ja and Gherardini FC (2008) Methods Mol Biol 431:213-21
2) Seshu J et al. (2004) Infect Immun 72(3): 1580-6
3) Marangoni A et al. (2006) World J Gastroenterol 12(19): 3077-81
4) Pancewicz Sa et al. (2007) Pol Merkur Lekarski 23(134): 141-4
5) Bitterman H (2009) Critical Care 13: 205
6) Zhukova AG and Sazontova TG (2005) Hypoxia Med J (3-4): 34-43
7) Wright CJ and Dennery PA (2009) Pediatr Res 66(1): 3-10
8) Assreuy et al. 1993; Colasanti and Suzuki 2000
9) Vanin et al. 2007, Yang and Loscalzo 2007

## Patentansprüche

1. Verfahren zur Verabreichung eines Gases an einen Menschen oder ein Säugetier, **dadurch gekennzeichnet, dass** in aufeinanderfolgenden Intervallen hypoxisches Gas alternierend mit hyperoxischem Gas verabreicht wird.

2. Verfahren nach Anspruch 1, **dadurch gekennzeichnet**, die Verabreichungsdauer des hypoxischen oder des hyperoxischen Gases 1 bis 20 Minuten, bevorzugt 2 bis 9 Minuten und besonders bevorzugt 3 bis 5 Minuten beträgt.

3. Verfahren nach einem der vorhergehenden Ansprüche, **dadurch gekennzeichnet, dass** die Verabreichungsdauer von hypoxischem und hyperoxischem Gas unterschiedlich lang ist.

4. Verfahren nach einem der vorhergehenden Ansprüche, **dadurch gekennzeichnet, dass** die eine Sauerstoffkonzentration im hypoxischen Gas 9 bis 15 Vol-% und die Sauerstoffkonzentration im hyperoxischen Gas 30 bis 40 Vol-% beträgt.

5. Verfahren nach einem der vorhergehenden Ansprüche, **dadurch gekennzeichnet, dass** die Pause zwischen der Verabreichung des hypoxischen und des hyperoxischen Gases nicht mehr als 20 Minuten, bevorzugt weniger als 10 Minuten und besonders bevorzugt weniger als 5 Minuten beträgt.

6. Verfahren nach einem der vorhergehenden Ansprüche, **dadurch gekennzeichnet, dass** die Verabreichung in Einheiten erfolgt, die aus mehreren aufeinanderfolgenden Verabreichungsintervallen von hypoxischem/hyperoxischem Gas besteht.

7. Verfahren nach einem der vorhergehenden Ansprüche, **dadurch gekennzeichnet, dass** 2 bis 10 Einheiten pro Woche verabreicht werden.

8. Verwendung des Verfahrens gemäß Anspruch 1 zur Leistungssteigerung, zur kosmetischen Gewichtsreduktion, zum Muskelaufbau, zur Verringerung oder Steigerung des Appetits, gegen Müdigkeit, zur Verlangsamung von Alterungsprozessen der Haut und/oder zur Verjüngung der Haut.
